# EUROPEAN PATENT SPECIFICATION

(11) **EP 2 914 342 B1**
(45) Date of publication and mention of the grant of the patent: **30.08.2017**
(21) Application number: 13801478.2
(22) Date of filing: 24.10.2013
(51) Int. Cl.: A61N 7/02

(54) **MEDICAL APPARATUS FOR DETERMINING A MAXIMUM ENERGY MAP**
MEDIZINISCHES GERÄT ZUM BESTIMMEN DER MAXIMALEN ENERGIEKARTE
APPAREIL MÉDICAL POUR DÉTERMINER UNE CARTE D'ÉNERGIE MAXIMALE

(30) Priority: 05.11.2012 EP 12191213
(43) Date of publication of application: 09.09.2015
(73) Proprietor: Koninklijke Philips N.V., 5656 AE Eindhoven (NL)
(72) Inventor: NURMILAUKAS, Kirsi Ilona, 5656 AE Eindhoven (NL); YLIHAUTALA, Mika Petri, 5656 AE Eindhoven (NL); VUORINEN, Reko Tapio, 5656 AE Eindhoven (NL); ENHOLM, Julia Kristiana, 5656 AE Eindhoven (NL); TÖLÖ, Jaakko Juhani, 5656 AE Eindhoven (NL)
(74) Representative: Cohen, Julius Simon
(86) International application number: PCT/EP2013/072277
(87) International publication number: WO 2014/067844

(56) References cited:
- WO-A1-2007/047247
- WO-A1-2010/029479
- NICK TODD ET AL: "Combining thermal and ultrasound modeling techniques for improved monitoring of MR guided HIFU treatments", AIP CONFERENCE PROCEEDINGS, 1 January 2010 (2010-01-01), pages 375-378, XP055058355, ISSN: 0094-243X, DOI: 10.1063/1.3367185
- ARORA D ET AL: "Nonlinear and model predictive control of thermal dose in high temperature therapies", PROCEEDINGS OF THE 2003 AMERICAN CONTROL CONFERENCE. ACC. DENVER, CO, JUNE 4 - 6, 2003; [AMERICAN CONTROL CONFERENCE], NEW YORK, NY : IEEE, US, vol. 2, 4 June 2003 (2003-06-04), pages 1200-1205, XP010663950, DOI: 10.1109/ACC.2003.1239751 ISBN: 978-0-7803-7896-4
- A. PAYNE ET AL: "Optimization of HIFU Treatments for Use in Model Predictive Control", AIP CONFERENCE PROCEEDINGS, 1 January 2009 (2009-01-01), pages 170-174, XP055058359, ISSN: 0094-243X, DOI: 10.1063/1.3131406

## Description

### TECHNICAL FIELD

The invention relates to high intensity focused ultrasound, in particular to the determination of a maximum sonication energy.

### BACKGROUND OF THE INVENTION

Ultrasound from a focused ultrasonic transducer can be used to selectively treat regions within the interior of the body. Ultrasonic waves are transmitted as high energy mechanical vibrations. These vibrations induce tissue heating as they are damped, and they can also lead to cavitation. Both tissue heating and cavitation can be used to destroy tissue in a clinical setting. However, heating tissue with ultrasound is easier to control than cavitation. Ultrasonic treatments can be used to ablate tissue and to kill regions of cancer cells selectively. This technique has been applied to the treatment of uterine fibroids, and has reduced the need for hysterectomy procedures.

To selectively treat tissue, a focused ultrasonic transducer can be used to focus the ultrasound on a particular treatment or target volume. The transducer is typically mounted within a medium, such as degassed water, that is able to transmit ultrasound. Actuators are then used to adjust the position of the ultrasonic transducer and thereby adjust the tissue region that is being treated.

Focused ultrasonic transducers also typically have multiple transducer elements, wherein the amplitude and/or phase of the transducer elements are controllable. In particular the phase of individual or groups of transducer elements is often controlled to control the location of the focus of the ultrasound. This enables the rapid adjustment location of the focus and the sequential sonication of different sonication points or locations. The tissue of a subject between the transducer and a sonication point is typically referred to as the near field region. The ultrasound travels through the near field region to the sonication volume. This intermediate tissue is also heated, although not as much as the sonication volume. When sonicating multiple sonication points the near field region of the different sonication points may overlap. Because a particular portion of the near field region may overlap it may be heated multiple times. To avoid overheating this overlapping near field region there may need to be delays between sonicating multiple sonication points and/or a reduction in sonication power.

United States patent US 8,016,757 B2 discloses a non-invasive temperature estimation technique for HIFU therapy using backscattered ultrasound. Ultrasound data is collected from a thermal source and a mass of tissue before initiating therapy to measure two parameters of the bio-heat transfer equation (BHTE). Once calibrated to the specific mass of the tissue and the specific thermal source the BHTE can be used to generate a temperature dependence curve calibrated to the thermal source and tissue, and spatio-temporal temperature maps, to facilitate therapy planning. The international application WO2010/029479 discloses a therapy system provided with a control module to provide an estimate of the induced heating prior to a deposit of energy.

### SUMMARY OF THE INVENTION

The invention provides for a medical apparatus and a computer program product in the independent claims. Embodiments are given in the dependent claims.

As will be appreciated by one skilled in the art, aspects of the present invention may be embodied as an apparatus or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the processor of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example a data may be retrieved over a modem, over the internet, or over a local area network. Computer executable code embodied on a computer readable medium may be transmitted using any appropriate medium, including but not limited to wireless, wireline, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a processor. 'Computer storage' or 'storage' is a further example of a computer-readable storage medium. Computer storage is any non-volatile computer-readable storage medium. In some embodiments computer storage may also be computer memory or vice versa.

A 'processor' as used herein encompasses an electronic component which is able to execute a program or machine executable instruction or computer executable code. References to the computing device comprising "a processor" should be interpreted as possibly containing more than one processor or processing core. The processor may for instance be a multi-core processor. A processor may also refer to a collection of processors within a single computer system or distributed amongst multiple computer systems. The term computing device should also be interpreted to possibly refer to a collection or network of computing devices each comprising a processor or processors. The computer executable code may be executed by multiple processors that may be within the same computing device or which may even be distributed across multiple computing devices.

Computer executable code may comprise machine executable instructions or a program which causes a processor to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances the computer executable code may be in the form of a high level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of apparatus (systems) and computer program products according to embodiments of the invention. It will be understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, and accelerometer are all examples of user interface components which enable the receiving of information or data from an operator.

A 'hardware interface' as used herein encompasses an interface which enables the processor of a computer system to interact with and/or control an external computing device and/or apparatus. A hardware interface may allow a processor to send control signals or instructions to an external computing device and/or apparatus. A hardware interface may also enable a processor to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bistable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

An 'ultrasound window' as used herein encompasses a window which is able to transmit ultrasonic waves or energy. Typically a thin film or membrane is used as an ultrasound window. The ultrasound window may for example be made of a thin membrane of BoPET (Biaxially-oriented polyethylene terephthalate).

In one aspect the invention provides for a medical apparatus comprising a high-intensity focused ultrasound system for sonicating a subject. The medical apparatus further comprises a processor for controlling the medical apparatus. The medical apparatus further comprises a memory for storing machine-executable instructions for execution by the processor. Execution of the instructions causes the processor to produce sonication data descriptive of a previous sonication of the subject to the high-intensity focused ultrasound system. The previous sonication data may be used to control the high-intensity focused ultrasound system for the previous sonication or it may be data descriptive of the location of the various sonication points and how they were sonicated with the high-intensity focused ultrasound system. The previous sonication data is essentially descriptive of how ultrasound entered and was focused in the subject. The previous sonication data may also contain times, durations and powers used for the previous sonication.

Execution of the instructions further cause the processor to construct a thermal property map of the subject using the previous sonication data and a thermoacoustic model. The thermal property map is spatially dependent and temporally dependent. That is to say the thermal property map maps out a thermal property spatially and is also time-dependent. For instance if the thermal property is the temperature or an energy density deposited into the subject over time the energy will dissipate and the temperature will return to a normal body temperature. Execution of the instructions further cause the processor to determine a maximum energy map using the thermoacoustic model and the thermal property map. The maximum energy is time-dependent. The maximum energy map is descriptive of the maximum energy that may be deposited into a volume of the subject. This maximum energy may be determined by performing the calculation with the thermoacoustic model and modeling effects of performing another sonication at spatially different positions.

Typically the maximum energy for a particular location may be limited by previous sonications because ultrasound passes through the subject on the way to the focal point of the high-intensity focused ultrasound system. In particular the near field region is a region between a transducer for the high-intensity focused ultrasound system and the focal point. By performing repeated sonications the near field region can become heated. It may be necessary to allow the sonication points to be sonicated at a later time to prevent from overheating the near field region. Execution of the instructions further causes the processor to receive a selection of the at least one sonication volume from a user interface. A sonication volume as used herein is a volume of the subject which is determined to be or selected to be sonicated. Essentially the operator of the medical apparatus can look at the maximum energy map and see which regions of the subject the operator can deposit energy into at the current time. By displaying this data spatially the operator may be able to more efficiently use the medical apparatus. Displaying the maximum energy map may identify regions which the operator can sonicate immediately or after a short period of time. This may reduce the downtime and the costs associated with high-intensity focused ultrasound systems.

In another embodiment the high-intensity focused ultrasound system comprises an ultrasound transducer operating for focusing ultrasound into a sonication volume. The sonication volume may be one of the at least one sonication volumes which were selected using the interface. The thermoacoustic model is operable for determining the thermal property map in a sonication region of a subject. The sonication region as used herein encompasses the region of the subject through which a measurable or significant quantity of ultrasound passes through the subject. This would include not just the sonication volume but also the near field region and the far field region. The thermoacoustic model is operable for determining a predicted thermal property map in a sonication region using the thermal property map and a predicted ultrasound beam geometry.

The ultrasound beam geometry may be determined using an ultrasound transducer model and the selection of at least one sonication volume from the user interface. That is to say that the effect of sonicating the at least one sonication volume can be predicted using the thermal property map and the thermoacoustic model. The predicted thermal property map is descriptive of the thermal property. The predicted thermal property map is spatially dependent. In some embodiments the thermal property map may also be time-dependent. The thermoacoustic model is operable for determining the maximum power for each sonication volume by limiting a maximum thermal property in the predicted thermal property map to a predetermined maximum value. For instance the thermoacoustic model may model the near field region and ensure that it is not overheated by multiple sonications.

In some embodiments instead of the thermal property map being determined for a sonication region it may be restricted to the near field region between the ultrasound transducer and a sonication volume. This may be beneficial in some embodiments because it may be easier to calculate the thermal property map in the near field region by assuming that the subject is homogeneous.

In another embodiment execution of the instructions further cause the processor to calculate an energy intensity map using the beam geometry, the maximum power and a thermoacoustic model. Execution of the instructions further causes the processor to display the energy intensity map on the display. An energy intensity map as used herein is a spatial mapping which estimates the energy density in the beam path of the high-intensity focused ultrasound system. In this embodiment both the energy intensity map and the maximum energy map are displayed on the display. As an alternative only the energy intensity map may be displayed.

In another embodiment the ultrasound transducer comprises multiple ultrasound transducer elements. The multiple ultrasound transducer elements are controllable. By controllable it is understood herein that the phase and/or amplitude of electrical power supplied to the transducer elements may be controlled individually or as groups. The multiple ultrasound transducer elements are operable for adjusting an ultrasound beam path between the ultrasound transducer and the sonication volume. The predicted ultrasound beam geometry is descriptive of the ultrasound beam path. The thermoacoustic model is further operable for determining the predicted thermal property map by calculating adjustments to the ultrasound beam geometry using a beam path ultrasound transducer model. Essentially the path of the beam may be approximated or predicted using a model. Simple models such as the ray tracing method or assuming a geometric shape which represent the beam path are computationally easy ways of calculating this.

The predicted ultrasound beam geometry is a beam path of the ultrasound generated by the ultrasound transducer.

In another embodiment the adjustment of the ultrasound beam geometry is used in a sonication.

In another embodiment the medical instrument further comprises an ultrasound transducer actuator for moving the ultrasound transducer. The ultrasound transducer actuator determines a transducer location. Execution of the instructions further cause the processor to determine the predicted thermal property map by calculating adjustments to the transducer location and by using a translational ultrasound transducer model. In addition to being electronically steerable the ultrasound transducer may also be able to be moved spatially. This may be used to control where the ultrasound is focused.

Magnetic Resonance (MR) data is defined herein as being the recorded measurements of radio frequency signals emitted by atomic spins by the antenna of a Magnetic resonance apparatus during a magnetic resonance imaging scan. A Magnetic Resonance Imaging (MRI) image is defined herein as being the reconstructed two or three dimensional visualization of anatomic data contained within the magnetic resonance imaging data. This visualization can be performed using a computer.

MR thermometry data is defined herein as being the recorded measurements of radio frequency signals emitted by atomic spins by the antenna of a Magnetic resonance apparatus during a magnetic resonance imaging scan which contains information which may be used for magnetic resonance thermometry. Magnetic resonance thermometry functions by measuring changes in temperature sensitive parameters. Examples of parameters that may be measured during magnetic resonance thermometry are: the proton resonance frequency shift, the diffusion coefficient, or changes in the T1 and/or T2 relaxation time may be used to measure the temperature using magnetic resonance. The proton resonance frequency shift is temperature dependent, because the magnetic field that individual protons, hydrogen atoms, experience depends upon the surrounding molecular structure. An increase in temperature decreases molecular screening due to the temperature affecting the hydrogen bonds. This leads to a temperature dependence of the proton resonant frequency.

The proton density depends linearly on the equilibrium magnetization. It is therefore possible to determine temperature changes using proton density weighted images.

The relaxation times T1, T2, and T2-star (sometimes written as T2*) are also temperature dependent. The reconstruction of T1, T2, and T2-star weighted images can therefore be used to construct thermal or temperature maps.

The temperature also affects the Brownian motion of molecules in an aqueous solution. Therefore pulse sequences which are able to measure diffusion coefficients such as a pulsed diffusion gradient spin echo may be used to measure temperature.

One of the most useful methods of measuring temperature using magnetic resonance is by measuring the proton resonance frequency (PRF) shift of water protons. The resonant frequency of the protons is temperature dependent. As the temperature changes in a voxel the frequency shift will cause the measured phase of the water protons to change. The temperature change between two phase images can therefore be determined. This method of determining temperature has the advantage that it is relatively fast in comparison to the other methods. The PRF method is discussed in greater detail than other methods herein. However, the methods and techniques discussed herein are also applicable to the other methods of performing thermometry with magnetic resonance imaging.

In another embodiment the medical apparatus further comprises a magnetic resonance imaging system for acquiring thermal magnetic resonance data from an imaging zone. The sonication region is within the imaging zone. Execution of the instructions further causes the processor to repeatedly acquire the magnetic resonance thermal data from the sonication region. Execution of the instructions further cause the processor to reconstruct a temperature change rate map using the repeatedly acquired magnetic resonance thermal data.

In another embodiment the magnetic resonance imaging system is used to acquire magnetic resonance data which is used for guiding the high-intensity focused ultrasound system. For instance medical images may be registered to the location of the subject and also displayed on the display.

In another embodiment execution of the instructions causes the processor to display the temperature change rate map on the display. This embodiment may be beneficial because the temperature change rate map may indicate properties of the tissue of the subject useful for deciding on where to sonicate.

In another embodiment execution of the instructions further causes the processor to modify the thermoacoustic model using the temperature change rate map. For instance the thermoacoustic model may assume that energy or temperature is dissipated at a particular location at a determined rate. By actually measuring the rate at which temperature changes the thermoacoustic model may be updated or corrected for the specific subject.

In another embodiment execution of the instructions further causes the processor to receive a sonication energy. Execution of the instructions further cause the processor to determine a cool down time map using the sonication energy and the thermoacoustic model. The cool down time is spatially dependent and descriptive of when a chosen sonication volume can be sonicated at the sonication energy. A chosen sonication volume as used herein is defined to be an arbitrary volume which has been selected to be sonicated. The cool down time map is time dependent. Execution of the instructions further causes the processor to display the cool down time map on the display. The cool down time map is displayed on the user interface before the selection of at least one sonication volume is received from the user interface. The sonication energy may for instance be predetermined, it may be input from the user interface, or received from a treatment plan or other instructions.

The sonication power as used herein is the power concentrated into the sonication volume.

In another embodiment the cool down time map is also determined using a changing of the beam path for the high-intensity focused ultrasound system. For instance the beam path may be altered such that the cool down time is displayed for a particular region in the map.

In another embodiment execution of the instructions further causes the processor to receive a sonication duration. The maximum energy map is expressed as a maximum power map. Instead of displaying the energy map the maximum energy map is instead displayed in terms of the maximum power that the operator can direct into a particular volume of the subject at a given time. The sonication duration may be received through a user interface, it may be predetermined, or may be received through instructions such as a treatment plan.

In another embodiment execution of the instructions further causes the processor to generate sonication commands using the selection of at least one sonication volume. Execution of the instructions further cause the processor to control the high-intensity focused ultrasound system to sonicate the at least one sonication volume using the sonication commands.

In another embodiment execution of the instructions further causes the processor to determine an updated maximum power map using the thermal property map, the sonication commands and the thermoacoustic model. The updated maximum energy map is time dependent. It may also be a spatially dependent mapping.

Execution of the instructions further causes the processor to display the updated energy map on the display. Execution of the instructions further cause the processor to receive an updated selection of at least one sonication volume from the user interface.

In another embodiment the thermal property is the temperature.

In another embodiment the thermal property is the thermal dose.

In another embodiment the thermal property is the energy density.

In another aspect the invention provides for a computer program product comprising machine-executable instructions for execution by a processor controlling a medical apparatus. The medical apparatus comprises a high-intensity focused ultrasound system for sonicating a subject. Execution of the instructions cause the processor to receive previous sonication data descriptive of a previous sonication of the subject by the high-intensity focused ultrasound system. Execution of the instructions further cause the processor to construct a thermal property map of the subject using the previous sonication data and a thermoacoustic model. The thermal property map is descriptive of a thermal property. The thermal property map is spatially and temporally dependent. Execution of the instructions further causes the processor to determine a maximum energy map using the thermoacoustic model. The maximum energy is time dependent. The maximum energy may also be spatially dependent. Execution of the instructions further causes the processor to display the maximum energy map on a display. Execution of the instructions further causes the processor to receive a selection of at least one sonication volume from a user interface. There is also described a method of operating a medical apparatus. The medical apparatus comprises a high-intensity focused ultrasound system for sonicating a subject. The method comprises the step of receiving previous sonication data descriptive of a previous sonication of the subject by the high-intensity focused ultrasound system. The method further comprises the step of constructing a thermal property map of the subject using the previous sonication data and a thermoacoustic model. The thermal property map is descriptive of a thermal property. The thermal property map is spatially and temporally dependent. The method further comprises the step of determining a maximum energy map using the thermoacoustic model. The maximum energy is time dependent. The method further comprises the step of displaying the maximum energy map on a display. The method further comprises the step of receiving a selection of at least one sonication volume from a user interface.

It is understood that one or more of the aforementioned embodiments of the invention may be combined as long as the combined embodiments are not mutually exclusive.

### BRIEF DESCRIPTION OF THE DRAWINGS

In the following preferred embodiments of the invention will be described, by way of example only, and with reference to the drawings in which:
Fig.1 shows a block diagram which illustrates a method;
Fig. 2 shows a block diagram which illustrates another exemplary method;
Fig. 3 shows a block diagram which illustrates another exemplary method;
Fig. 4 shows a block diagram which illustrates an alternative method;
Fig. 5 illustrates a medical instrument according to an embodiment of the invention;
Fig. 6 shows a portion of Fig. 5, and
Fig. 7. illustrates a medical instrument according to a further embodiment of the invention; and
Fig. 8. illustrates a medical instrument according to a further embodiment of the invention.

### DETAILED DESCRIPTION OF THE EMBODIMENTS

Like numbered elements in these figures are either equivalent elements or perform the same function. Elements which have been discussed previously will not necessarily be discussed in later figures if the function is equivalent.

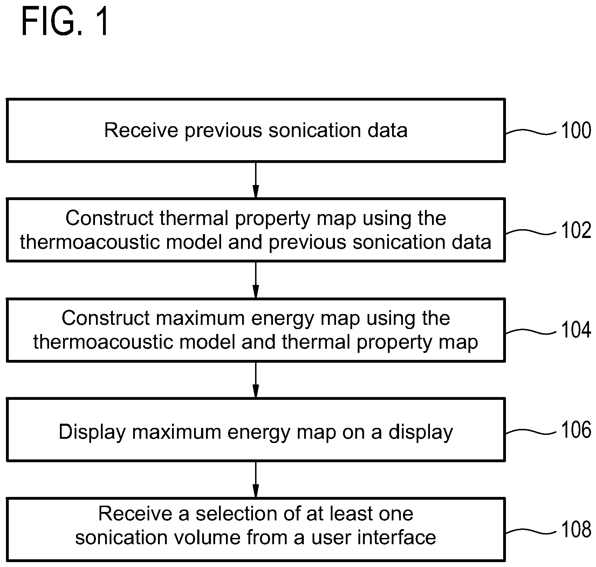

Fig. 1 shows a block diagram which illustrates a method. First in step 100 previous sonication data is received. The previous sonication data is descriptive of a previous sonication of the subject by a high-intensity focused ultrasound system. Next in step 102 a thermal property map is constructed of the subject using the previous sonication data and a thermoacoustic model. The thermal property map is descriptive of a thermal property. The thermal property map is spatially dependent and temporally dependent. Next in step 104 a maximum energy map is determined or constructed using the thermoacoustic model and the thermal property map. The maximum energy is time dependent. Next in step 106 the maximum energy map is displayed on a display. Then finally in step 108 a selection of at least one sonication volume is received from a user interface. The user interface may for instance be displayed on the display with the maximum energy map.

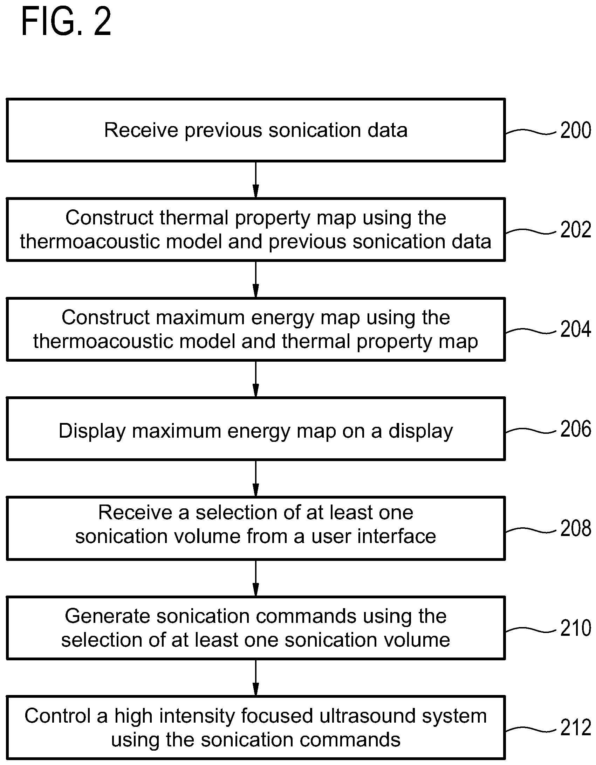

Fig. 2 shows a block diagram which illustrates another exemplary method. Steps 200-208 of this method are equivalent to steps 100-108 of the method shown in Fig. 1. First in step 200 previous sonication data is received. Next in step 202 a thermal property map is constructed using the thermoacoustic model and previous sonication data. Next in step 204 a maximum energy map is constructed using the thermoacoustic model and a thermal property map. Next in step 206 the maximum energy map is displayed on a display. Then in step number 208 a selection of at least one sonication volume is received from a user interface. Next in step 210 a set of sonication commands is generated using the selection of at least one sonication volume. The sonication commands are commands which are operable for causing the high-intensity focused ultrasound to sonicate the selected at least one sonication volume. Then finally in step 212 the high-intensity focused ultrasound system is controlled using the sonication commands in order to sonicate the at least one sonication volume.

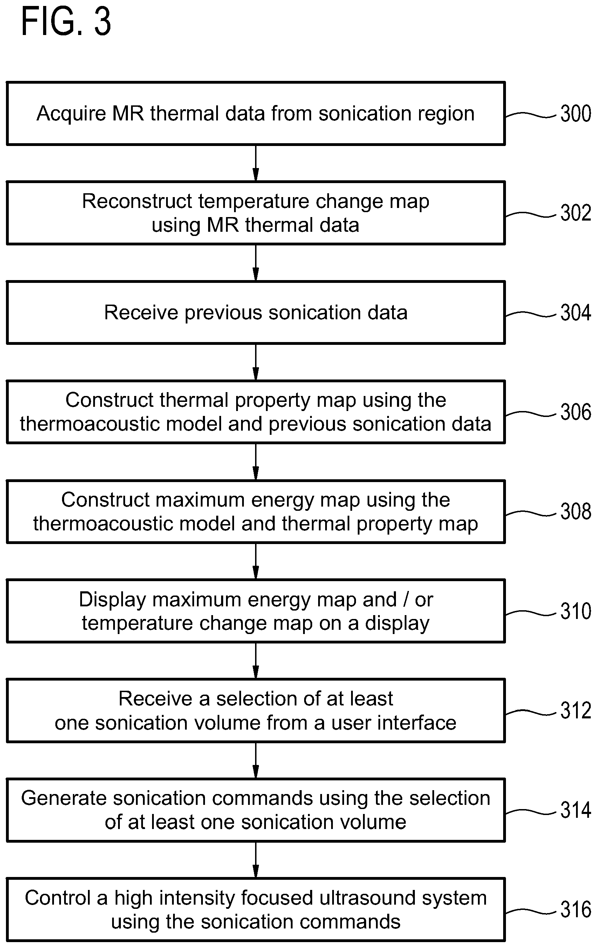

Fig. 3 shows a flow diagram which illustrates another exemplary method. First in step 300 magnetic resonance thermal data is acquired from a sonication region using the magnetic resonance imaging system. Next in step 302 a temperature change map is reconstructed using the magnetic resonance imaging thermal data. Next in step 304 previous sonication is received. The magnetic resonance imaging thermal data may be acquired during the previous sonication. Next in step 306 a thermal property map is constructed using the thermoacoustic model and the previous sonication data. Next in step 308 a maximum energy map is constructed using the thermoacoustic model and the thermal property map. Next in step 310 the maximum energy map and/or the temperature change map is displayed on a display. Next in step 312 a selection of at least one sonication volume is received from a user interface. Next in step 314 sonication commands are generated using the selection of at least one sonication volume. Then finally in step 316 a high-intensity focused ultrasound system is controlled using the sonication commands. This causes the high-intensity focused ultrasound system to sonicate the at least one sonication volume that was selected.

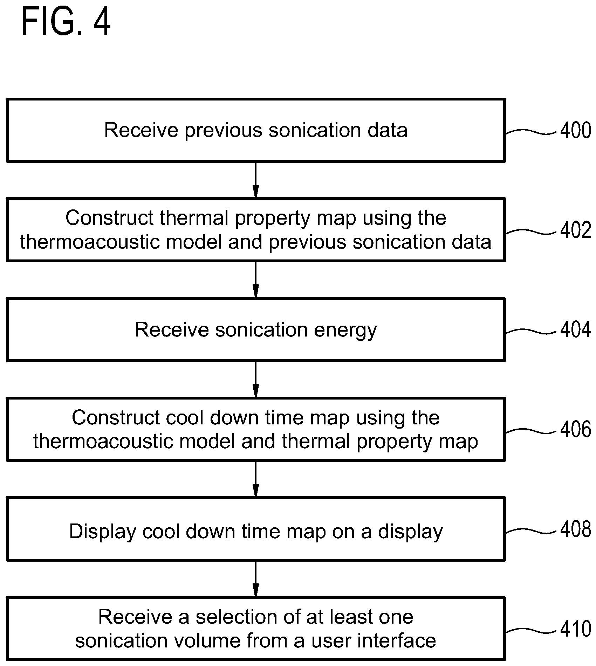

Fig. 4 shows a flow diagram of an alternative method. First in step 400 previous sonication data is received. Next in step 402 a thermal property map is constructed using the thermoacoustic model and the previous sonication data. Next in step 404 a sonication energy is received. Then in step 406 a cool down time map is constructed using the thermoacoustic model and the thermal property map. Then in step 408 the cool down time map is displayed on a display. Then finally in step 410 a selection of at least one sonication volume is received from the user interface.

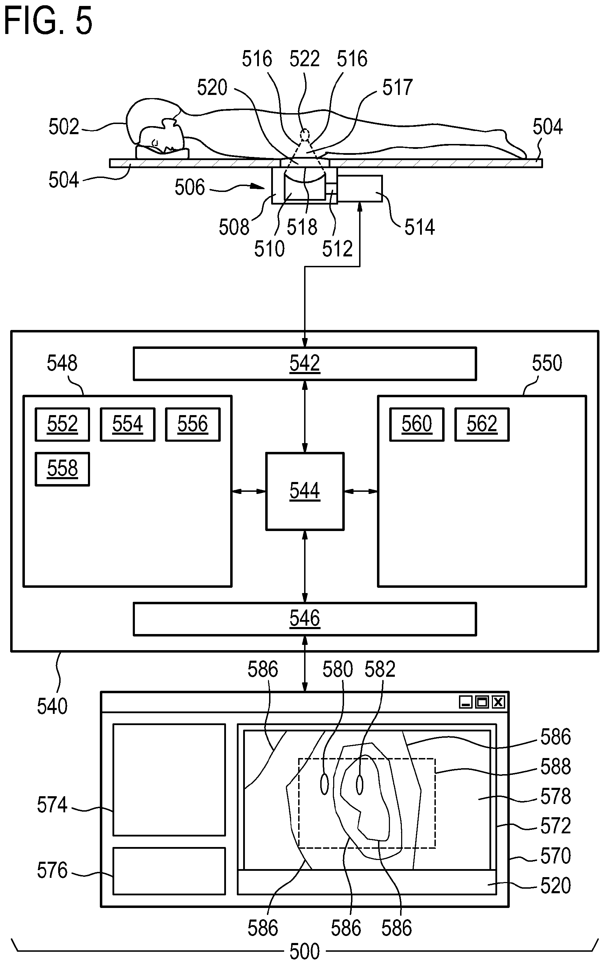

Fig. 5 illustrates an example of a medical apparatus 500. A subject 502 is shown as reposing on a subject support 504. The medical apparatus 500 comprises a high-intensity focused ultrasound system 506. The high-intensity focused ultrasound system comprises 506 a fluid-filled chamber 508. Within the fluid-filled chamber 508 is an ultrasound transducer 510. Although it is not shown in this figure the ultrasound transducer 510 comprises multiple ultrasound transducer elements each capable of generating an individual beam of ultrasound. This may be used to steer the location of a sonication volume 522 electronically by controlling the phase and/or amplitude of alternating electrical current supplied to each of or groups of the ultrasound transducer elements. Point 522 represents the adjustable focus of the medical apparatus 500.

The ultrasound transducer 510 is connected to a mechanism 512 which allows the ultrasound transducer 510 to be repositioned mechanically. The mechanism 512 is connected to a mechanical actuator 514 which is adapted for actuating the mechanism 512. The mechanical actuator 512 also represents a power supply for supplying electrical power to the ultrasound transducer 510. In some embodiments the power supply may control the phase and/or amplitude of electrical power to individual ultrasound transducer elements.

The ultrasound transducer 510 generates ultrasound which is shown as following the path 516. The ultrasound 516 goes through the fluid-filled chamber 508 and through an ultrasound window 518. In this embodiment the ultrasound then passes through a gel pad 520. The gel pad 520 is not necessarily present in all embodiments but in this embodiment there is a recess in the subject support 504 for receiving a gel pad 520. The gel pad 520 helps couple ultrasonic power between the transducer 510 and the subject 502. After passing through the gel pad 520 the ultrasound 516 passes through a near field region 517 of the subject 502 and then is focused to a sonication volume 522 or target zone.

The sonication volume 522 may be moved through a combination of mechanically positioning the ultrasonic transducer 510 and electronically steering the position of the sonication volume 522.

The computer 540 further comprises a processor 544, a user interface 546, computer storage 548, and computer memory 550. The hardware interface 542 enables the processor 544 to send and receive commands and data in order to control the functioning of the medical apparatus 500. The processor 544 is further connected to the user interface 546, the computer storage 548, and the computer memory 550.
The computer storage 548 is shown as containing previous sonication data 552. The computer storage 548 is further shown as containing a thermal property map 554. The computer storage 548 is further shown as containing a maximum energy map 556. The computer storage 548 is also shown as containing a selection of the sonication volume.

The computer memory 550 is shown as containing a control module 560. The control module contains computer-executable code which enables the processor 544 to control the operation and function of the medical apparatus 500. The computer memory 550 is also shown as containing a thermoacoustic model 562. The thermoacoustic model 562 contains computer-executable code 544 which enables the processor to calculate the thermal property map from the previous sonication data 552 and the maximum energy map 556 from the thermal property map 554.

The user interface 546 is connected to a display which is displaying a graphical user interface 570. The graphical user interface 570 has several different regions. There is a graphic display region 572 for displaying mappings and if available medical image data. There is a data display region 574 where messages and data can be displayed to the operator and there is also a tool selection region 576. The tool selection region 576 contains tools which allow the operator to modify the interface in order to enter data and/or to select sonication volumes for sonication. The data display region for instance may display data relevant to selected sonication volumes. For instance it may display the available power which may be used to sonicate a selected sonication volume, the maximum energy which may be deposited into a sonication volume, and/or the cool down time.

The graphic display region 572 shows graphically the results of the thermoacoustic model 562. In this region 572 there are two selected sonication volumes, a first sonication volume 580 and a second sonication volume 582. There are contour lines 586 which represent a mapping displayed in the display region 572. The mapping 586 may represent a maximum energy map, a thermal property map, an energy intensity map, a temperature map, a temperature change rate map and a cool down time map. One or more of these various mappings may be displayed. They may be for instance displayed using contour lines or other graphical means such as colored images. If displayed they may also be displayed in a semi-transparent or transparent fashion such that more than one layer can be displayed. For instance the available power and the cool down time may both be displayed at the same time. For instance if the region in the contour surrounding sonication volume 582 is at a lower maximum energy than sonication volume 580 the user may select to sonicate volume 580 first in some instances. The dashed line 588 indicates an optional indicator which shows the available region for sonication.

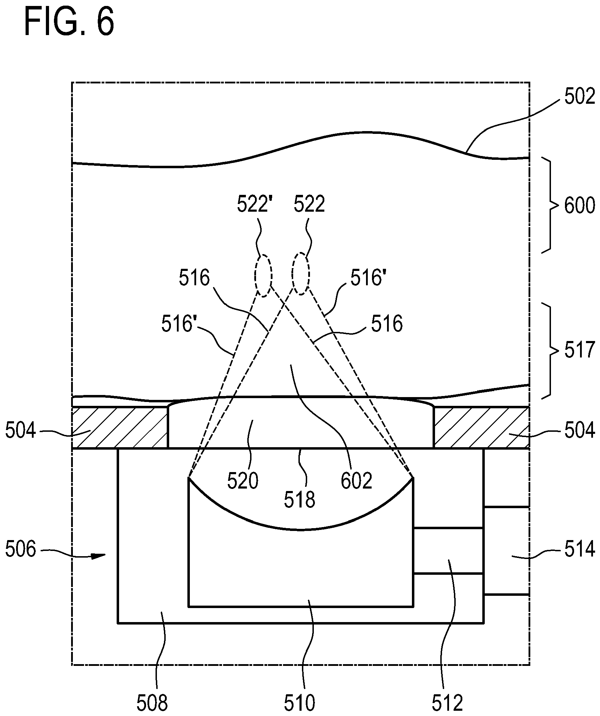

Fig. 6 shows an enlarged portion of Fig. 5. The high-intensity focused ultrasound system 506 and a portion of the subject 502 is shown. In this example the sonication point is shown at two different locations, 522 and 522'. These are two sonication points which were performed sequentially and were changed using a change in the electronic focusing of the ultrasound transducer 510. The dashed lines 516 show the approximate path of ultrasound within a cone focusing on the sonication volume 522. The dashed lines marked 516' show the cone of approximate travel of ultrasound being focused into the sonication volume 522'. It can be seen that these two regions of ultrasound 516, 516' overlap in region 602. The overlapping region 602 would be heated when both sonication volumes 522, 522' are sonicated. The overlapping region 602 would be heated more than the ultrasound in the regions which are not overlapping. This Fig. illustrates that it is beneficial to use the thermoacoustic model to construct a thermal property map which is descriptive of the change in the thermal properties of the subject 502 after the points 522 and 522' have been sonicated. Also marked on Fig. 6 is the near field region 517 between the sonication points 522, 522' and the transducer 510 and also the far field region 600 which is beyond the sonication points 522, 522'.

In Fig. 6, the sonication of the two sonication volumes 522, 522' was accomplished by using electronic steering. An overlapping region 602 may also be caused by mechanically repositioning the transducer 510. The example presented in Fig. 6 therefore applies also to mechanical positioning of the sonication volumes 522, 522'.

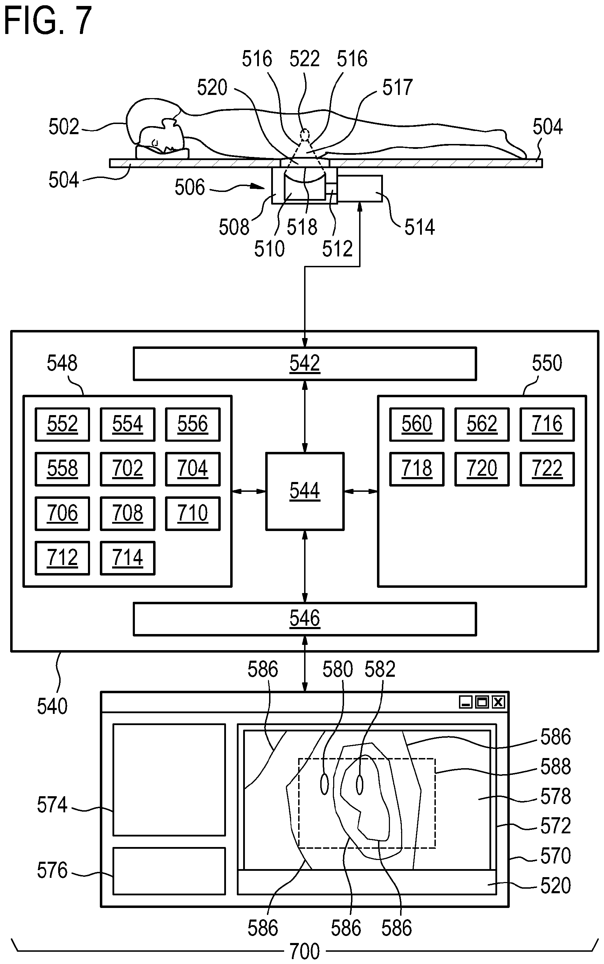

Fig. 7 shows a further embodiment of the medical instrument 700. The embodiment shown in Fig. 7 is similar to that shown in Fig. 5 except there are additional software components. The computer storage 548 is shown as additionally containing an energy intensity map. The computer storage 548 is also shown as additionally containing an ultrasound beam path 704. The ultrasound beam path 704 is descriptive of a calculated beam path for the ultrasound transducer 510. The computer storage 548 is further shown as containing a received sonication energy 706. The computer storage 548 is shown as further containing a calculated cool down time map 708 for the region 578. The computer storage 548 is further shown as containing a sonication duration 710 which has been received. The computer storage 548 is further shown as containing a maximum power map 712. The computer storage 548 is further shown as containing sonication commands 714 for controlling the high-intensity focused ultrasound system 506 to sonicate the sonication points 580 and 582.

The computer memory 550 is shown as further containing an energy intensity map calculation module 716. The energy intensity map calculation module 716 contains computer-executable code which enables the processor 544 to calculate the energy intensity map 702 using the previous sonication data 552. The computer memory 550 is further shown as containing beam path prediction module 718. The beam path prediction module 718 contains computer-executable code which enables the processor 544 to calculate the ultrasound beam path 704 which is used in beam shaping for optimizing the cool down time map, the energy intensity map, the maximum power map and the maximum energy map. The computer memory 550 is shown as further containing a maximum power map calculation module.

The maximum power map calculation module 720 contains computer-executable code which enables the processor 544 to calculate the maximum power map 712 using the sonication duration 710 and the maximum energy map 556. Essentially the sonication duration 710 is used to make a transformation from energy to power. The computer memory 550 is further shown as containing a sonication command generation module 722. The sonication command generation module 722 contains computer-executable code which enables the processor to calculate the sonication commands 714 necessary to sonicate the selected sonication volumes 580 and 582. In some embodiments there may be beam shaping in which case the beam path predication module 718 is also used by the module 722.

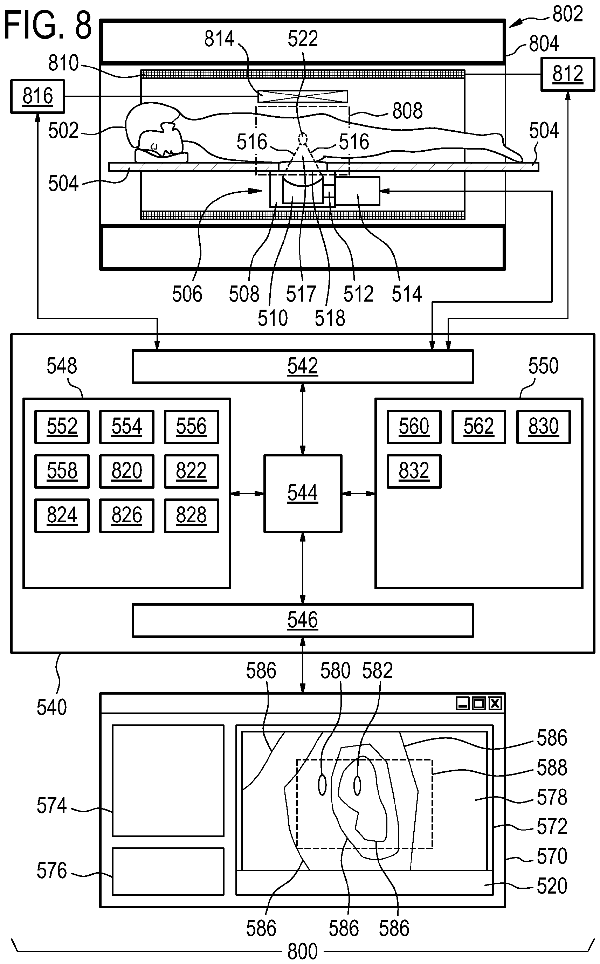

Fig. 8 shows a further example of a medical apparatus 800. The medical apparatus 800 is similar to the medical apparatuss 500 shown in Fig. 5 and 700 shown in Fig. 7. In this embodiment there is a magnetic resonance imaging system 802 for acquiring thermal magnetic resonance data. The magnetic resonance imaging system comprises a magnet 804. The magnet 804 is a cylindrical type superconducting magnet with a bore 806 through the center of it. In various embodiments the mechanical actuator/power supply 514 is located outside or inside of the bore 806 of the magnet 804.

The magnet has a liquid helium cooled cryostat with superconducting coils. It is also possible to use permanent or resistive magnets. The use of different types of magnets is also possible for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils. Within the bore 806 of the cylindrical magnet there is an imaging zone 808 where the magnetic field is strong and uniform enough to perform magnetic resonance imaging.

Within the bore 806 of the magnet there is also a set of magnetic field gradient coils 810 which are used for acquisition of magnetic resonance data to spatially encode magnetic spins within the imaging zone 808 of the magnet 804. The magnetic field gradient coils are connected to a magnetic field gradient coil power supply 812. The magnetic field gradient coils 810 are intended to be representative. Typically magnetic field gradient coils contain three separate sets of coils for spatially encoding in three orthogonal spatial directions. A magnetic field gradient power supply 812 supplies current to the magnetic field gradient coils 810. The current supplied to the magnetic field coils is controlled as a function of time and may be ramped or pulsed.

Adjacent to the imaging zone 808 is a radio-frequency coil 814 for manipulating the orientations of magnetic spins within the imaging zone 808 and for receiving radio transmissions from spins also within the imaging zone. The radio-frequency coil may contain multiple coil elements. The radio-frequency coil may also be referred to as a channel or an antenna. The radio-frequency coil 814 is connected to a radio frequency transceiver 816. The radio-frequency coil 814 and radio frequency transceiver 816 may be replaced by separate transmit and receive coils and a separate transmitter and receiver. It is understood that the radio-frequency coil 814 and the radio-frequency transceiver 816 are representative. The radio-frequency coil 814 is intended to also represent a dedicated transmit antenna and a dedicated receive antenna. Likewise the transceiver 816 may also represent a separate transmitter and receivers.

The computer storage 548 is shown as containing a pulse sequence 820. A pulse sequence as used herein is a sequence of commands performed at different times which enable a magnetic resonance imaging system 802 to acquire magnetic resonance data 822. The computer storage 548 is shown as containing thermal magnetic resonance data 822 that has been acquired using the magnetic resonance imaging system 802. The computer storage 548 is further shown as containing a thermal property map 824, a temperature change rate map 826, and/or the medical image 828 that was reconstructed from the magnetic resonance data 822.

The computer memory 550 is shown as containing an image reconstruction module 830. The image reconstruction module 830 contains computer-executable code which enables the processor 544 to construct the thermal property map 824, the temperature change rate map 826, and/or the medical image 828 from the thermal magnetic resonance data 522.

The computer memory 550 is also shown as containing a thermoacoustic model correction module 832. This is an optional module 832 that may use the temperature change rate map 826 to correct the thermoacoustic model 562.

The magnetic resonance imaging system 802 may also be used for guiding the high-intensity focused ultrasound system 506. For instance through normal magnetic resonance imaging the magnetic resonance imaging system 802 may be used to identify anatomical landmarks within the subject 502 to identify the location of the sonication points 880 and 882.

With HIFU therapy modality, there are several factors that affect treatment efficiency or safety. These factors are often dependent on the patient tissue properties or chosen treatment (sonication) parameters, and are not known to the user during treatment, or at least their spatial distribution is unknown. The system can, however, measure some parameters describing these properties. If spatial or treatment cell-dependent information about such parameters were available during a treatment event to the user, she/he could use the data to optimize the treatment.

Embodiments may be operable to visualize one or more of the following:
- Treatment Cell dependent cooldown time, indicated for each planned cell
- Any parametrization, visualized as a map of e.g. contours or colors, describing the treatment parameters, tissue properties or prediction of success or danger of future treatments. It can be for example:
   - Cooldown/heatup rate map
   - Relaxation time map (e.g. Time constant of fat, measured from T2 weighted image)
   - Cooldown time map for each cell type
   - Available power or energy or maximum treatment cell size map
   - Energy or intensity density map
- Available treatment area, taking into account the energy deposited in previously performed treatment events
- Combined visualization of temperature maps obtained by different measurement methods

Embodiments may provide relevant information that is acquired from the target during usage, to the user that is controlling the usage. The user can then use the information to optimize and adjust the usage according to the information that is given. If the information is not available to the user, the treatment outcome, efficiency or safety may be worse that what it could be when using the invention.

Within HIFU treatment, there can be areas of tissue, where several sonications have been recently performed, and considerable build up of heat exists. Such areas should be avoided from further sonication until enough time has elapsed and it is safe to sonicate again. The user has no exact knowledge about which areas to avoid. Several aspects of this invention can be used to give such information to the user.

Embodiments may be operable to parameterize information that the system has gathered during the treatment, and visualize it to the user in a form that enables the user to easily understand it and take advantage of the data in treatment planning.

Embodiments may be operable to gather information about the patient tissue properties, recovery from previous treatment history, readiness for future treatment event or available options for future treatment events. Embodiments may be operable to then visualize the information to the user, in a form that is easy to understand and useful to the user for optimizing future treatment.

The rate, at which tissue cools down after a heating event, or heats up during a heating event, depends for example on tissue type, its vascularity, heat diffusion from other areas, and the surrounding tissues. User (and system) can make assumptions of the cooldown or heat up rate for each tissue type and use these assumptions to calculate predictions for future treatment events. However, the system can alternatively determine the actual temperature behavior in each tissue, if it measures the temperature maps in sufficient intervals. The determination can be done for example by fitting exponential or other suitable functions to the measured data. Based on such maps, it can construct 2D or 3D maps of temperature decay or heatup rate for each tissue. The heatup rate map can take the energy or intensity, incident on that tissue during treatment, into account, to produce more reliable maps. The system can then visualize such maps to the user, for example with contours or colors. The user can then use the visualized information to detect differences in, for example, temperature cooldown after HIFU sonication, and to select suitable cooldown times for each tissue. The system can also automatically use the measured cooldown rate maps when calculating cooldown times for planned future treatment events. Or, based on a visualized, measured heatup rate map, the user can estimate how much energy is needed to perform treatment in each particular location. Also, the system can use the heatup rate maps to give a suggestion about the suitable treatment parameters for the future treatment events.

The cool down time that is considered sufficient before starting a new treatment event (for example, sonication with HIFU device), can depend on the planned energy of the future treatment event, and the history of past treatment events that may have created heat accumulation in tissues in the beam path. The position dependence can be calculated and visualized to the user (e.g. with 2D or 3D contours or colors). The user can use the visualizations to deduce, which treatment positions are available for future treatment soonest, and which positions should yet not be treated for a while, due to too large heat accumulation in the beam path. The user can then plan new treatment events in the area that is indicated as "short" cool down time area, thereby reducing total treatment duration. The cool down time maps can also be calculated and visualized separately for treatments of different energies, for example per cell type, to enable optimization of treatment with the different energy options.

The power or energy, available or recommendable, for treatment can depend on the treatment position. The position dependence may be caused for example by heat accumulation from previous treatments, information offered by previous sonications about suitable energy or power need for close-by treatment positions, or existence of sensitive organs in the energy propagation path. The system can calculate the available (if strict rules exist for example in shaping the beam to avoid marked regions) or recommendable (if no strict rules exist, but system has information that advices not to use too high power intensity or energy over a certain region) power intensity, or maximum power intensity, or energy, or maximum energy, to be used in treatment in the treatment area, as a 2D or 3D map, and visualize it to the user, for example by contours or colors. The user can utilize the information to choose suitable power intensity or energy or cell size for each treatment event. The system can also automatically restrict available power or energy for a treatment event, based on the calculated map and planned treatment location.

The maximum treatment cell size, available or recommendable, for treatment can depend on the treatment position. The position dependence may be caused for example by heat accumulation from previous treatments, information offered by previous sonications about suitable energy or power need for close-by treatment positions, existence of sensitive organs in the energy propagation path, transducer mechanical movement range restrictions combined with available deflection ranges for each cell type. The system can calculate the available (if strict rules exist for example in shaping the beam to avoid marked regions) or recommendable (if no strict rules exist, but system has information that advices not to use too large cell sizes over a certain region) maximum treatment cell size, to be used in treatment in the treatment area, as a 2D or 3D map, and visualize it to the user, for example by colors or contours. The user can utilize the information to choose suitable (or most likely to be successful) treatment cell size for each treatment event.

Embodiments may be operable to have knowledge about the regions through which energy or intensity travels during a treatment event. The system can estimate the energy density in the beam path in several ways. For the HIFU treatment, it can be obtained for example with thermoacoustic simulations, or with some simpler model, assuming for example conic sonication energy distribution and dividing simply the total energy by the area of the cone in the plane being investigated. Attenuation of tissues in which the energy travels, can optionally be taken into account. Such models work best in the near field region, since then neither the (unknown) attenuation of tissue, nor the phase coherence, building up at focal area, have a large effect on the energy distribution. In the far field, pre-calculated simulated energy (cell) and deflection dependent field models can be used. These are especially suitable for Sonalleve HIFU feedback cells, where the energy at focus is approximately the same for each sonication of the same cell size and axial deflection, and the far field beam shape can be well approximated by a pre-simulated shape.

These methods of calculating the energy density of all past treatment events can be combined to construct an energy density map of all previous treatment events and visualized to the user, as a 2D or 3D map of for example contours or colors. The time elapsed since each past treatment event can be taken into account by for example using a sliding average of the past energy/intensity densities, or by assuming or measuring a time constant of decay for each tissue separately and using that on the energy/intensity for each past treatment event.

The user can then compare the beam, or similar energy or intensity map of a planned treatment event, with the energy or intensity density map from previous treatment events to see if the planned treatment event is going to cause energy deposition in the areas that already received energy or intensity, and probably not yet fully cooled down.

Embodiments may have software operable to visualize the available treatment area (ATA) to the user. The 3D ATA does not currently take the treatment history into account. The system could detect which areas of the treatment area have received significant energy recently, and indicate to the user, which focal areas require energy transmit through areas that are not safe for sonication. The ATA would then be time dependent, releasing more areas for treatment, as time passes and the tissues cool down.

For volumetric sonications, the cool down time can depend on cell size. The larger cells require larger total energies than smaller cells. Therefore, they should have longer waiting times between sonications, to enable sufficient cooling of the tissue in between sonications. Also, the dose accumulation increases in a complex exponential manner with increased temperature; therefore, cool down times can be chosen such that initial remaining temperature from previous sonications before starting a sonication cell, is smaller with larger cells, and larger with smaller cells. The cool down time can be predicted for each cell before starting a sonication. Some cells (also non-volumetric cells) may also have no, or minimal, overlap of energy distribution in the energy propagation path, with the previously sonicated cells, thereby allowing instant sonication after a previous cell. The cool down times can then be quite different for different future cells, but the user has no knowledge about the position or cell type dependence unless it is indicated by the system. The essential feature of this aspect of the innovation is to calculate cell dependent cool down times, and visualize them to the user. This aspect of the invention could be implemented for example by associating and visualizing a cooldown time with each cell. For example, in a cell list, or in an image with planned cells, the cooldown time would be visible close to the visualization or other representation of the cell.

One example of usage of some embodiments is with HIFU using beam shaping. The beam shaping algorithm can take the actual sonication power into account, and determine which transducer elements to switch off or reduce in intensity so that the allowed intensity or energy exposure of an organ avoidance region is not exceeded. With the approach of this invention, the system can calculate (prior to a sonication) a (2D or 3D) map, indicating which maximum energy or power can be used in each focal position, so that the intensity or energy limits of the organ avoidance regions are not exceeded, and other conditions set for using beam shaping are fulfilled (e.g. maximum number of switched off elements). Without such a map, the user has no information in the planning stage about what power he/she can use in which part of the treatment area, and whether a certain part of the treatment area has too low available powers or energies so that the clinically successful treatment outcome is unlikely.

Another example of usage by some embodiments would be with any HIFU treatment, proposing a suitable energy or intensity for a sonication. The energy need at a certain treatment location is expected to be close to the energy need at a location that is close to it, since the similar tissues usually need similar energies, to get ablated, even though some variance exists e.g. due to differences in tissues in the beam path. Farther tissues may also have different vascularity and other tissue properties causing different energy needs for ablation. The deeper lying tissues usually require higher energies than tissues closer to skin, to be ablated, since the attenuation of the tissues between focus and transducer reduces the energy arriving at focal region. The software could investigate the energies or intensities used with previous successful sonications that are sufficiently close to the planned treatment location, for example by taking a weighted (by proximity) average of the energies of these sonications, and determine the probable "successful" power intensity or energy for the next sonication, at each available sonication location and construct a (2D or 3D) map of "recommendable" power. This could be determined separately for the different cell sizes. If only depth dependence in energy or power need is considered, the recommended power could alternatively be obtained simply by assuming some predefined attenuation alfa for the tissue in question, i.e. the sonication power or energy E would depend only on sonication depth with a law E = Eref *e ^{2*alfa*(Depth-Depthref)}. The model would require a reference energy Eref at a reference depth Depthref. This could be obtained for example by using average (over some patient population) needed energy at some sonication depth, or by using some (or several) past sonications within the current treatment. This information, obtained in whichever mentioned method, could be visualized as a (2D or 3D) map of recommended power as colors or contours.

Different aspects of the invention can be applied on any imaging modality that is able to produce temperature maps at a rate that is sufficiently quick for using the methods. For example, MR guided HIFU treatment modality. Some aspects of the invention can be used also in a broader context such as for visualizing the available treatment area and/or visualizing the cell dependent cooling time.

Embodiments may be operable to visualize the available treatment area. This can also be applied to any modality that visualizes the (2D or 3D) available area for treatment or any other action, to take into account previous history of treatment or any other action, and to modify the visualized available area to indicate which areas should be avoided for further treatment or other action, until certain conditions, set for starting the treatment or other action, are fulfilled.

Embodiments may be operable to be applied to other modalities requiring cool down times, or other waiting times between treatment or action events, when the length of the required waiting time depends on the previous treatment or action history. The appropriate waiting time could be calculated for each planned future treatment or action event, and visualized or indicated for the user, to enable optimization of treatment or action order.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 500: medical instrument
- 502: subject
- 504: subject support
- 506: high intensity focused ultrasound system
- 508: fluid filled chamber
- 510: ultrasound transducer
- 512: mechanism
- 514: mechanical actuator/power supply
- 516: path of ultrasound
- 517: near field region
- 518: ultrasound window
- 520: gel pad
- 522: sonication volume
- 540: computer system
- 542: hardware interface
- 544: processor
- 546: user interface
- 548: computer storage
- 550: computer memory
- 552: previous sonication data
- 554: thermal property map
- 556: maximum energy map
- 558: selection of sonication volume
- 560: control module
- 562: thermoacoustic model
- 570: user interface
- 572: graphic display region
- 574: data display region
- 576: tool selection region
- 578: sonication region
- 580: first sonication volume
- 582: second sonication volume
- 584: available sonication region
- 586: contour line
- 588: available sonication region
- 600: far field region
- 602: overlapping region
- 700: medical instrument
- 702: energy intensity map
- 704: ultrasound beam path
- 706: sonication energy
- 708: cool down time map
- 710: sonication duration
- 712: maximum power map
- 714: sonication commands
- 716: energy intensity map calculation module
- 718: beam path prediction module
- 720: maximum power map calculation module
- 722: sonication command generation module
- 800: medical apparatus
- 802: magnetic resonance imaging system
- 804: magnet
- 806: bore of magnet
- 808: imaging zone
- 810: magnetic field gradient coils
- 812: magnetic field gradient coils power supply
- 814: radio-frequency coil
- 816: transceiver
- 820: pulse sequence
- 822: thermal magnetic resonance data
- 824: thermal property map
- 826: temperature change rate map
- 828: medical image
- 830: image reconstruction module
- 832: thermoacoustic model correction module

## Claims

1. A medical apparatus (500, 700, 800) comprising:
- a high intensity focused ultrasound system (506) for sonicating a subject (502), wherein the high intensity focused ultrasound system comprises an ultrasound transducer (510) operable for focusing ultrasound into a sonication volume (522),
- a display (572);
- a user interface (570);
- a processor (544) adapted to control the medical apparatus,
- a memory (550) adapted to store machine executable instructions (560, 562) for execution by the processor, wherein execution of the instructions cause the processor to:
- receive (100, 200, 304) previous sonication data (522) descriptive of a previous sonication of the subject by the high intensity focused ultrasound system;
- construct (102, 202, 306) a thermal property map (554) of the subject using the previous sonication data and a thermoacoustic model (562), wherein the thermal property map is descriptive of a thermal property, wherein the thermal property map is spatially dependent and temporally dependent, wherein the thermal property is any one of the following: temperature, thermal dose, and an energy density, wherein the thermoacoustic model is operable for determining the thermal property map in a sonication region (578) of the subject, wherein the thermoacoustic model is further operable for determining a predicted thermal property map in the sonication region using a predicted ultrasound beam geometry (704) and the thermal property map; wherein the predicted thermal property map is descriptive of the thermal property, wherein the predicted thermal property map is spatially dependent, wherein the thermoacoustic model is further operable for determining the maximum power for each sonication volume by limiting a maximum thermal property in the predicted thermal property map to a predetermined maximum value;
- determine (104, 204, 308) a maximum energy map (556) using the thermoacoustic model and the thermal property map, wherein the maximum energy is time dependent, wherein the maximum energy map is descriptive of a maximum energy which may be deposited into a volume of the subject to prevent overheating of a near field region of the subject being a region between the ultrasound transducer (510) and the sonication volume (522);
- display (106, 206, 310) the maximum energy map on the display (572); and
- receive (108, 208, 312) a selection of at least one sonication volume (580, 582) from the user interface (570), the at least one sonication volume being a volume of the subject which is selected to be sonicated.

2. The medical apparatus of claim1, wherein execution of the instructions causes the processor to:
- calculate an energy intensity map (702) using the beam geometry, the maximum power, and the thermoacoustic model, wherein the energy intensity map is a spatial mapping which estimates an energy density in the beam path of the high-intensity focused ultrasound system; and
- display the energy intensity map on the display.

3. The medical apparatus of claim 1 or 2, wherein the ultrasound transducer comprises multiple ultrasound transducer elements, wherein the multiple ultrasound transducer elements are controllable, wherein the multiple ultrasound transducer elements are operable for adjusting an ultrasound beam path between the ultrasound transducer and the sonication volume, wherein the predicted ultrasound beam geometry is descriptive of the ultrasound beam path, wherein the thermoacoustic model is further operable for determining the predicted thermal property map by calculating adjustments to the ultrasound beam geometry using a beam path ultrasound transducer model (718).

4. The medical apparatus of claim 1, 2, or 3, wherein the medical apparatus further comprises an ultrasound transducer actuator (512, 514) adapted to move the ultrasound transducer, wherein the ultrasound transducer actuator is adapted to determine a transducer location,
wherein execution of the instructions further cause the processor to determine the predicted thermal property map by calculating adjustments to the transducer location and by using a translational ultrasound transducer model.

5. The medical apparatus of any one of claims 1 through 4, wherein the medical apparatus further comprises a magnetic resonance imaging system (802) adapted to acquire thermal magnetic resonance data (822) from an imaging zone (808), wherein the sonication region is within the imaging zone, wherein execution of the instructions further causes the processor to:
- repeatedly acquire (300) the magnetic resonance thermal data from the sonication region; and
- reconstruct (302) a temperature change rate map (826) using the repeatedly acquired magnetic resonance thermal data.

6. The medical instrument of claim 5, wherein execution of the instructions causes the processor to display the temperature change rate map on the display.

7. The medical apparatus of claim 5 or 6, wherein execution of the instructions causes the processor to modify the thermoacoustic model using the temperature change rate map.

8. The medical apparatus of any one of the preceding claims, wherein execution of the instructions further cause the processor to:
- receive a sonication energy (706);
- determine a cool down time map (708) using the sonication energy and the thermoacoustic model, wherein the cool down time is spatially descriptive of when a chosen sonication volume can be sonicated at the sonication energy, wherein the cool down map is time dependent; and
- display the cool down time map on the display, wherein the cool down time map is displayed on the display before the selection of at least one sonication volume is received from the user interface.

9. The medical apparatus of any one of the preceding claims, wherein execution of the instructions further causes the processor to receive a sonication duration (710), and wherein the maximum energy map is expressed as a maximum power map (712).

10. The medical apparatus of any one of the preceding claims, wherein execution of the instructions causes the processor to:
- generate (210) sonication commands (714) using the selection of at least one sonication volume; and
- control (220) the high intensity focused ultrasound system to sonicate the at least one sonication volume using the sonication commands.

11. The medical apparatus of claim 10, wherein execution of the instructions further cause the processor to:
- determine an updated maximum power map using the thermal property map, the sonication commands, and the thermoacoustic model; , wherein the updated maximum energy map is time dependent;
- display the updated energy map on the display; and
- receive an updated selection of at the least one sonication volume from the user interface.

12. A computer program product comprising machine executable instructions (560, 562) for execution by a processor (544) controlling a medical apparatus, wherein the medical apparatus comprises a high intensity focused ultrasound system (506) for sonicating a subject (502), wherein the high intensity focused ultrasound system comprises an ultrasound transducer (510) operable for focusing ultrasound into a sonication volume (522), wherein the medical apparatus further comprises a display (572), a user interface (570) and a memory adapted to store the machine executable instructions, wherein execution of the instructions cause the processor to:
- receive (100, 200, 304) previous sonication data (522) descriptive of a previous sonication of the subject by the high intensity focused ultrasound system;
- construct (102, 202, 306) a thermal property map (554) of the subject using the previous sonication data and a thermoacoustic model (562), wherein the thermal property map is descriptive of a thermal property, wherein the thermal property map is spatially dependent and temporally dependent, wherein the thermal property is any one of the following: temperature, thermal dose, and an energy density, wherein the thermoacoustic model is operable for determining the thermal property map in a sonication region (578) of the subject, wherein the thermoacoustic model is further operable for determining a predicted thermal property map in the sonication region using a predicted ultrasound beam geometry (704) and the thermal property map; wherein the predicted thermal property map is descriptive of the thermal property, wherein the predicted thermal property map is spatially dependent, wherein the thermoacoustic model is further operable for determining the maximum power for each sonication volume by limiting a maximum thermal property in the predicted thermal property map to a predetermined maximum value;;
- determine (104, 204, 308) a maximum energy map (556) using the thermoacoustic model, wherein the maximum energy is time dependent, wherein the maximum energy map is descriptive of a maximum energy which may be deposited into a volume of the subject to prevent overheating of a near field region of the subject being a region between the ultrasound transducer (510) and the sonication volume (522);
- display (106, 206, 310) the maximum energy map on the display (572); and
- receive (108, 208, 312) a selection of at least one sonication volume (580, 582) from the user interface (570), the at least one sonication volume being a volume of the subject which is selected to be sonicated.

## Patentansprüche

1. Medizinisches Gerät (500, 700, 800), umfassend:
- ein mit hochintensiv fokussiertem Ultraschall arbeitendes System (506) zum Beschallen eines Subjekts (502), wobei das mit hochintensiv fokussiertem Ultraschall arbeitende System einen Ultraschallwandler (510) umfasst, der betriebsfähig ist, um Ultraschall in ein Beschallungsvolumen (522) zu fokussieren,
- eine Anzeige (572);
- eine Benutzerschnittstelle (570);
- einen Prozessor (544), der dafür ausgelegt ist, das medizinische Gerät zu steuern,
- einen Speicher (550), der dafür ausgelegt ist, maschinenausführbare Anweisungen (560, 562) zur Ausführung durch den Prozessor zu speichern, wobei die Ausführung der Anweisungen den Prozessor veranlasst zum:
- Empfangen (100, 200, 304) von vorhergehenden Beschallungsdaten (522), die eine vorhergehende Beschallung des Subjekts durch das mit hochintensiv fokussiertem Ultraschall arbeitende System beschreiben;
- Konstruieren (102, 202, 306) einer thermischen Eigenschaftskarte (554) des Subjekts unter Verwendung der vorhergehenden Beschallungsdaten und eines thermoakustischen Modells (562), wobei die thermische Eigenschaftskarte eine thermische Eigenschaft beschreibt, wobei die thermische Eigenschaftskarte räumlich abhängig und zeitlich abhängig ist, wobei die thermische Eigenschaft eines von Folgendem ist: Temperatur, thermische Dosis und eine Energiedichte, wobei das thermoakustische Modell nutzbar ist, um die thermische Eigenschaftskarte in einer Beschallungsregion (578) des Subjekts zu bestimmen, wobei das thermoakustische Modell weiterhin nutzbar ist, um unter Verwendung einer vorhergesagten Ultraschallstrahlenbündelgeometrie (704) und der thermischen Eigenschaftskarte eine vorhergesagte thermische Eigenschaftskarte in der Beschallungsregion zu bestimmen; wobei die vorhergesagte thermische Eigenschaftskarte die thermische Eigenschaft beschreibt, wobei die vorhergesagte thermische Eigenschaftskarte räumlich abhängig ist, wobei das thermoakustische Modell weiterhin nutzbar ist, um die maximale Leistung für jedes Beschallungsvolumen zu bestimmen, indem eine maximale thermische Eigenschaft in der vorhergesagten thermischen Eigenschaftskarte auf einen vorgegebenen Maximalwert begrenzt wird;
- Bestimmen (104, 204, 308) einer maximalen Energiekarte (556) unter Verwendung des thermoakustischen Modells und der thermischen Eigenschaftskarte, wobei die maximale Energie zeitabhängig ist, wobei die maximale Energiekarte eine maximale Energie beschreibt, die in ein Volumen des Subjekts eingebracht werden kann, um eine Überhitzung einer Nahfeldregion des Subjekts zu vermeiden, bei der es sich um eine Region zwischen dem Ultraschallwandler (510) und dem Beschallungsvolumen (522) handelt;
- Anzeigen (106, 206, 310) der maximalen Energiekarte auf der Anzeige (572); und
- Empfangen (108, 208, 312) einer Auswahl von mindestens einem Beschallungsvolumen (580, 582) von der Benutzerschnittstelle (570), wobei das mindestens eine Beschallungsvolumen ein Volumen des Subjekts ist, das als zu beschallen ausgewählt wurde.

2. Medizinisches Gerät nach Anspruch 1, wobei die Ausführung der Anweisungen den Prozessor veranlasst zum:
- Berechnen einer Energieintensitätskarte (702) unter Verwendung der Strahlenbündelgeometrie, der maximalen Leistung und des thermoakustischen Modells, wobei die Energieintensitätskarte eine räumliche Abbildung ist, die eine Energiedichte in dem Strahlengang des mit hochintensiv fokussiertem Ultraschall arbeitenden Systems schätzt; und
- Anzeigen der Energieintensitätskarte auf der Anzeige.

3. Medizinisches Gerät nach Anspruch 1 oder 2, wobei der Ultraschallwandler mehrere Ultraschallwandlerelemente umfasst, wobei die mehreren Ultraschallelemente steuerbar sind, wobei die mehreren Ultraschallwandlerelemente nutzbar sind, um einen Ultraschallstrahlengang zwischen dem Ultraschallwandler und dem Beschallungsvolumen anzupassen, wobei die vorhergesagte Ultraschallstrahlenbündelgeometrie den Ultraschallstrahlengang beschreibt, wobei das thermoakustische Modell weiterhin nutzbar ist, um die vorhergesagte thermische Eigenschaftskarte zu bestimmen, indem Anpassungen an der Ultraschallstrahlenbündelgeometrie unter Verwendung eines Strahlengang-Ultraschallwandlermodells (718) berechnet werden.

4. Medizinisches Gerät nach Anspruch 1, 2 oder 3, wobei das medizinische Gerät weiterhin einen Ultraschallwandler-Aktor (512, 514) umfasst, der dafür ausgelegt ist, den Ultraschallwandler zu bewegen, wobei der Ultraschallwandler-Aktor dafür ausgelegt ist, einen Wandlerort zu bestimmen, wobei die Ausführung der Anweisungen den Prozessor weiterhin veranlasst, die vorhergesagte thermische Eigenschaftskarte zu bestimmen, indem Anpassungen an dem Wandlerort berechnet werden und indem ein translationales Ultraschallwandlermodell verwendet wird.

5. Medizinisches Gerät nach einem der Ansprüche 1 bis 4, wobei das medizinische Gerät weiterhin ein Magnetresonanzbildgebungssystem (802) umfasst, das dafür ausgelegt ist, thermische Magnetresonanzdaten (822) aus einer Bildgebungszone (808) zu erfassen, wobei die Beschallungsregion innerhalb der Bildgebungszone liegt, wobei die Ausführung der Anweisungen den Prozessor weiterhin veranlasst zum:
- wiederholten Erfassen (300) der thermischen Magnetresonanzdaten aus der Beschallungsregion; und
- Rekonstruieren (302) einer Temperaturveränderungsgeschwindigkeitskarte (826) unter Verwendung der wiederholt erfassten thermischen Magnetresonanzdaten.

6. Medizinisches Instrument nach Anspruch 5, wobei die Ausführung der Anweisungen den Prozessor veranlasst, die Temperaturveränderungsgeschwindigkeitskarte auf der Anzeige anzuzeigen.

7. Medizinisches Gerät nach Anspruch 5 oder 6, wobei die Ausführung der Anweisungen den Prozessor veranlasst, das thermoakustische Modell unter Verwendung der Temperaturveränderungsgeschwindigkeitskarte zu modifizieren.

8. Medizinisches Gerät nach einem der vorhergehenden Ansprüche, wobei die Ausführung der Anweisungen den Prozessor weiterhin veranlasst zum:
- Empfangen einer Beschallungsenergie (706);
- Bestimmen einer Herunterkühlungszeitkarte (708) unter Verwendung der Beschallungsenergie und des thermoakustischen Modells, wobei die Herunterkühlungszeit räumlich beschreibt, wann ein gewähltes Beschallungsvolumen mit der Beschallungsenergie beschallt werden kann, wobei die Herunterkühlungskarte zeitabhängig ist; und
- Anzeigen der Herunterkühlungszeitkarte auf der Anzeige, wobei die Herunterkühlungszeitkarte auf der Anzeige angezeigt wird, bevor die Auswahl von mindestens einem Beschallungsvolumen von der Benutzerschnittstelle empfangen wird.

9. Medizinisches Gerät nach einem der vorhergehenden Ansprüche, wobei die Ausführung der Anweisungen den Prozessor weiterhin dazu veranlasst, eine Beschallungsdauer (710) zu empfangen, und wobei die maximale Energiekarte als eine maximale Leistungskarte (712) ausgedrückt wird.

10. Medizinisches Gerät nach einem der vorhergehenden Ansprüche, wobei die Ausführung der Anweisungen den Prozessor veranlasst zum:
- Erzeugen (210) von Beschallungsbefehlen (714) unter Verwendung der Auswahl von mindestens einem Beschallungsvolumen; und
- Steuern (220) des mit hochintensiv fokussiertem Ultraschall arbeitenden Systems zum Beschallen des mindestens einen Beschallungsvolumens unter Verwendung der Beschallungsbefehle.

11. Medizinisches Gerät nach Anspruch 10, wobei die Ausführung der Anweisungen den Prozessor weiterhin veranlasst zum:
- Bestimmen einer aktualisierten maximalen Leistungskarte unter Verwendung der thermischen Eigenschaftskarte, der Beschallungsbefehle und des thermoakustischen Modells; wobei die aktualisierte maximale Energiekarte zeitabhängig ist;
- Anzeigen der aktualisierten Energiekarte auf der Anzeige; und
- Empfangen einer aktualisierten Auswahl des mindestens einen Beschallungsvolumens von der Benutzerschnittstelle.

12. Computerprogrammprodukt umfassend maschinenausführbare Anweisungen (560, 562) zur Ausführung durch einen Prozessor (544) zur Steuerung eines medizinischen Geräts, wobei das medizinische Gerät ein mit hochintensiv fokussiertem Ultraschall arbeitendes System (506) zum Beschallen eines Subjekts (502) umfasst, wobei das mit hochintensiv fokussiertem Ultraschall arbeitende System einen Ultraschallwandler (510) umfasst, der betriebsfähig ist, um Ultraschall in ein Beschallungsvolumen (522) zu fokussieren, wobei das medizinische Gerät weiterhin eine Anzeige (572), eine Benutzerschnittstelle (570) und einen Speicher umfasst, der dafür ausgelegt ist, die maschinenausführbaren Anweisungen zu speichern, wobei die Ausführung der Anweisungen den Prozessor veranlasst zum:
- Empfangen (100, 200, 304) von vorhergehenden Beschallungsdaten (522), die eine vorhergehende Beschallung des Subjekts durch das mit hochintensiv fokussiertem Ultraschall arbeitende System beschreiben;
- Konstruieren (102, 202, 306) einer thermischen Eigenschaftskarte (554) des Subjekts unter Verwendung der vorhergehenden Beschallungsdaten und eines thermoakustischen Modells (562), wobei die thermische Eigenschaftskarte eine thermische Eigenschaft beschreibt, wobei die thermische Eigenschaftskarte räumlich abhängig und zeitlich abhängig ist, wobei die thermische Eigenschaft eines von Folgendem ist: Temperatur, thermische Dosis und eine Energiedichte, wobei das thermoakustische Modell nutzbar ist, um die thermische Eigenschaftskarte in einer Beschallungsregion (578) des Subjekts zu bestimmen, wobei das thermoakustische Modell weiterhin nutzbar ist, um unter Verwendung einer vorhergesagten Ultraschallstrahlenbündelgeometrie (704) und der thermischen Eigenschaftskarte eine vorhergesagte thermische Eigenschaftskarte in der Beschallungsregion zu bestimmen; wobei die vorhergesagte thermische Eigenschaftskarte die thermische Eigenschaft beschreibt, wobei die vorhergesagte thermische Eigenschaftskarte räumlich abhängig ist, wobei das thermoakustische Modell weiterhin nutzbar ist, um die maximale Leistung für jedes Beschallungsvolumen zu bestimmen, indem eine maximale thermische Eigenschaft in der vorhergesagten thermischen Eigenschaftskarte auf einen vorgegebenen Maximalwert begrenzt wird;
- Bestimmen (104, 204, 308) einer maximalen Energiekarte (556) unter Verwendung des thermoakustischen Modells, wobei die maximale Energie zeitabhängig ist, wobei die maximale Energiekarte eine maximale Energie beschreibt, die in ein Volumen des Subjekts eingebracht werden kann, um eine Überhitzung einer Nahfeldregion des Subjekts zu vermeiden, bei der es sich um eine Region zwischen dem Ultraschallwandler (510) und dem Beschallungsvolumen (522) handelt;
- Anzeigen (106, 206, 310) der maximalen Energiekarte auf der Anzeige (572); und
- Empfangen (108, 208, 312) einer Auswahl von mindestens einem Beschallungsvolumen (580, 582) von der Benutzerschnittstelle (570), wobei das mindestens eine Beschallungsvolumen ein Volumen des Subjekts ist, das als zu beschallen ausgewählt wurde.

## Revendications

1. Appareil médical (500, 700, 800) comprenant :
- un système ultrasonore focalisé haute intensité (506) pour opérer une sonication sur un sujet (502), dans lequel le système ultrasonore focalisé haute intensité comprend un transducteur ultrasonore (510) exploitable pour focaliser des ultrasons dans un volume de sonication (522),
- un afficheur (572) ;
- une interface utilisateur (570) ;
- un processeur (544) adapté pour commander l'appareil médical,
- une mémoire (550) adaptée pour stocker des instructions exécutables par machine (560, 562) pour une exécution par le processeur, dans lequel l'exécution des instructions amène le processeur à :
- recevoir (100, 200, 304) des données de sonication antérieures (522) descriptives d'une sonication antérieure du sujet par le système ultrasonore focalisé haute intensité ;
- construire (102, 202, 306) une carte de propriété thermique (554) du sujet à l'aide des données de sonication antérieures et d'un modèle thermoacoustique (562), dans lequel la carte de propriété thermique est descriptive d'une propriété thermique, dans lequel la carte de propriété thermique est spatialement dépendante et temporellement dépendante, dans lequel la propriété thermique est l'une quelconque parmi les suivantes : une température, une dose thermique et une densité d'énergie, dans lequel le modèle thermoacoustique est exploitable pour déterminer la carte de propriété thermique dans une région de sonication (578) du sujet, dans lequel le modèle thermoacoustique est en outre exploitable pour déterminer une carte de propriété thermique prédite dans la région de sonication à l'aide d'une géométrie de faisceau ultrasonore prédite (704) et de la carte de propriété thermique ; dans lequel la carte de propriété thermique prédite est descriptive de la propriété thermique, dans lequel la carte de propriété thermique prédite est spatialement dépendante, dans lequel le modèle thermoacoustique est en outre exploitable pour déterminer la puissance maximale pour chaque volume de sonication en limitant une propriété thermique maximale dans la carte de propriété thermique prédite à une valeur maximale prédéterminée ;
- déterminer (104, 204, 308) une carte d'énergie maximale (556) à l'aide du modèle thermoacoustique et de la carte de propriété thermique, dans lequel l'énergie maximale est dépendante du temps, dans lequel la carte d'énergie maximale est descriptive d'une énergie maximale qui peut être déposée dans un volume du sujet pour empêcher une surchauffe d'une région de champ proche du sujet qui est une région entre le transducteur ultrasonore (510) et le volume de sonication (522) ;
- afficher (106, 206, 310) la carte d'énergie maximale sur l'afficheur (572) ; et
- recevoir (108, 208, 312) une sélection d'au moins un volume de sonication (580, 582) à partir de l'interface utilisateur (570), l'au moins un volume de sonication étant un volume du sujet qui est sélectionné pour faire l'objet d'une sonication.

2. Appareil médical selon la revendication 1, dans lequel une exécution des instructions amène le processeur à :
- calculer une carte d'intensité d'énergie (702) à l'aide de la géométrie de faisceau, de la puissance maximale et du modèle thermoacoustique, dans lequel la carte d'intensité d'énergie est une cartographie spatiale qui estime une densité d'énergie dans le chemin de faisceau du système ultrasonore focalisé haute intensité ; et
- afficher la carte d'intensité d'énergie sur l'afficheur.

3. Appareil médical selon la revendication 1 ou 2, dans lequel le transducteur ultrasonore comprend de multiples éléments transducteurs ultrasonores, dans lequel les multiples éléments transducteurs ultrasonores sont commandables, dans lequel les multiples éléments transducteurs ultrasonores sont exploitables pour ajuster un chemin de faisceau ultrasonore entre le transducteur ultrasonore et le volume de sonication, dans lequel la géométrie de faisceau ultrasonore prédite est descriptive du chemin de faisceau ultrasonore, dans lequel le modèle thermoacoustique est en outre exploitable pour déterminer la carte de propriété thermique prédite en calculant des ajustements sur la géométrie de faisceau ultrasonore à l'aide d'un modèle transducteur ultrasonore de chemin de faisceau (718).

4. Appareil médical selon la revendication 1, 2, ou 3, dans lequel l'appareil médical comprend en outre un actionneur de transducteur ultrasonore (512, 514) adapté pour déplacer le transducteur ultrasonore, dans lequel l'actionneur de transducteur ultrasonore est adapté pour déterminer un emplacement de transducteur, dans lequel une exécution des instructions amène en outre le processeur à déterminer la carte de propriété thermique prédite en calculant des ajustements sur l'emplacement de transducteur et en utilisant un modèle de transducteur ultrasonore de translation.

5. Appareil médical selon l'une quelconque des revendications 1 à 4, dans lequel l'appareil médical comprend en outre un système d'imagerie par résonance magnétique (802) adapté pour acquérir des données de résonance magnétique thermiques (822) à partir d'une zone d'imagerie (808), dans lequel la région de sonication est au sein de la zone d'imagerie, dans lequel une exécution des instructions amène en outre le processeur à :
- acquérir de façon répétée (300) les données thermiques de résonance magnétique à partir de la région de sonication ; et
- reconstruire (302) une carte de cadence de changement de température (826) à l'aide des données thermiques de résonance magnétique acquises de façon répétée.

6. Instrument médical selon la revendication 5, dans lequel une exécution des instructions amène le processeur à afficher la carte de cadence de changement de température sur l'afficheur.

7. Appareil médical selon la revendication 5 ou 6, dans lequel une exécution des instructions amène le processeur à modifier le modèle thermoacoustique à l'aide de la carte de cadence de changement de température.

8. Appareil médical selon l'une quelconque des revendications précédentes, dans lequel une exécution des instructions amène en outre le processeur à :
- recevoir une énergie de sonication (706) ;
- déterminer une carte de temps de refroidissement (708) à l'aide de l'énergie de sonication et du modèle thermoacoustique, dans lequel le temps de refroidissement est spatialement descriptif du moment où un volume de sonication sélectionné peut faire l'objet d'une sonication à l'énergie de sonication, dans lequel la carte de refroidissement est dépendante du temps ; et
- afficher la carte de temps de refroidissement sur l'afficheur, dans lequel la carte de temps de refroidissement est affichée sur l'afficheur avant que la sélection d'au moins un volume de sonication soit reçue à partir de l'interface utilisateur.

9. Appareil médical selon l'une quelconque des revendications précédentes, dans lequel une exécution des instructions amène en outre le processeur à recevoir une durée de sonication (710), et dans lequel la carte d'énergie maximale est exprimée comme une carte de puissance maximale (712).

10. Appareil médical selon l'une quelconque des revendications précédentes, dans lequel une exécution des instructions amène le processeur à :
- générer (210) des ordres de sonication (714) à l'aide de la sélection d'au moins un volume de sonication ; et
- commander (220) le système ultrasonore focalisé haute intensité pour opérer une sonication sur l'au moins un volume de sonication à l'aide des ordres de sonication.

11. Appareil médical selon la revendication 10, dans lequel une exécution des instructions amène en outre le processeur à :
- déterminer une carte de puissance maximale mise à jour à l'aide de la carte de propriété thermique, des ordres de sonication et du modèle thermoacoustique ; dans lequel la carte d'énergie maximale mise à jour est dépendante du temps ;
- afficher la carte d'énergie mise à jour sur l'afficheur ; et
- recevoir une sélection mise à jour de l'au moins un volume de sonication à partir de l'interface utilisateur.

12. Produit-programme d'ordinateur comprenant des instructions exécutables par machine (560, 562) pour exécution par un processeur (544) commandant un appareil médical, dans lequel l'appareil médical comprend un système ultrasonore focalisé haute intensité (506) pour opérer une sonication sur un sujet (502), dans lequel le système ultrasonore focalisé haute intensité comprend un transducteur ultrasonore (510) exploitable pour focaliser des ultrasons dans un volume de sonication (522), dans lequel l'appareil médical comprend en outre un afficheur (572), une interface utilisateur (570) et une mémoire adaptée pour stocker les instructions exécutables par machine, dans lequel une exécution des instructions amène le processeur à :
- recevoir (100, 200, 304) des données de sonication antérieures (522) descriptive d'une sonication antérieure du sujet par le système ultrasonore focalisé haute intensité ;
- construire (102, 202, 306) une carte de propriété thermique (554) du sujet à l'aide des données de sonication antérieures et d'un modèle thermoacoustique (562), dans lequel la carte de propriété thermique est descriptive d'une propriété thermique, dans lequel la carte de propriété thermique est spatialement dépendante et temporellement dépendante, dans lequel la propriété thermique est l'une quelconque parmi les suivantes : une température, une dose thermique et une densité d'énergie, dans lequel le modèle thermoacoustique est exploitable pour déterminer la carte de propriété thermique dans une région de sonication (578) du sujet, dans lequel le modèle thermoacoustique est en outre exploitable pour déterminer une carte de propriété thermique prédite dans la région de sonication à l'aide d'une géométrie de faisceau ultrasonore prédite (704) et de la carte de propriété thermique ; dans lequel la carte de propriété thermique prédite est descriptive de la propriété thermique, dans lequel la carte de propriété thermique prédite est spatialement dépendante, dans lequel le modèle thermoacoustique est en outre exploitable pour déterminer la puissance maximale pour chaque volume de sonication en limitant une propriété thermique maximale dans la carte de propriété thermique prédite à une valeur maximale prédéterminée ;
- déterminer (104, 204, 308) une carte d'énergie maximale (556) à l'aide du modèle thermoacoustique , dans lequel l'énergie maximale est dépendante du temps, dans lequel la carte d'énergie maximale est descriptive d'une énergie maximale qui peut être déposée dans un volume du sujet pour empêcher une surchauffe d'une région de champ proche du sujet qui est une région entre le transducteur ultrasonore (510) et le volume de sonication (522) ;
- afficher (106, 206, 310) la carte d'énergie maximale sur l'afficheur (572) ; et
- recevoir (108, 208, 312) une sélection d'au moins un volume de sonication (580, 582) à partir de l'interface utilisateur (570), l'au moins un volume de sonication étant un volume du sujet qui est sélectionné pour faire l'objet d'une sonication.
